# EUROPEAN PATENT SPECIFICATION

(11) **EP 1 379 181 B1**
(45) Date of publication and mention of the grant of the patent: **10.12.2008**
(21) Application number: 02761986.5
(22) Date of filing: 29.03.2002
(51) Int. Cl.: A61B 17/34, A61M 29/02

(54) **RADIALLY EXPANDABLE DILATOR**
RADIAL AUSDEHNBARER DILATATOR
DILATATEUR EXTENSIBLE RADIALEMENT

(30) Priority: 16.04.2001 US 283637 P
(43) Date of publication of application: 14.01.2004
(73) Proprietor: CYTYC CORPORATION, Marlborough, MA 01752 (US)
(72) Inventor: HUNG, David, Belmont, CA 94002 (US); OLSEN, Phillip, M., Cupertino, CA 95014 (US); SCHULZ, Grace, San Carlos, CA 94070 (US); PATEL, Tina, J., San Carlos, CA 94070 (US)
(74) Representative: Tombling, Adrian George
(86) International application number: PCT/US2002/009587
(87) International publication number: WO 2002/083013

(56) References cited:
- EP-A- 0 093 101
- EP-A- 0 177 177
- WO-A-96/23536
- WO-A-99/17665
- WO-A-99/55384
- GB-A- 2 240 926

## Description

The present invention relates to a device for dilating an opening and passageway within a mammalian body, more specifically, the present invention relates to a device that dilates an opening in a mammalian body and internally receives a medical instrument so that the received instrument can pass through the device and be positioned within the body without causing the patient discomfort.

### Background of the Invention

Many medical procedures require that a medical instrument, such as a catheter, be introduced into an opening in the body for various reasons. These procedures may also require that the instrument be advanced through the opening during the procedure. In order to introduce the medical instrument, with the patient experiencing the least possible amount of discomfort, many practitioners attempt to dilate the opening before or during the introduction of the instrument. The devices used to dilate these openings are commonly referred to as dilators. As used herein, the term "opening" includes, but is not limited to, natural openings to body passageways, such as ductal openings in nipples, and surgically created openings.

A conventional dilator is normally sized so that it fits within and can extend through an internal lumen in a medical instrument that needs to be positioned in a body opening. Traditionally, the outer diameter of the dilator is smaller than the inner diameter of the medical instrument so that the dilator can be removed after the opening has been properly dilated and the medical instrument seated. When properly positioned within the medical instrument, a distal (leading) end of the dilator will extend beyond a distal end of the medical instrument so that the dilator enters the body opening first and begins to gradually dilate (expand) the body opening so that it can accept the outer diameter of the medical instrument.

Some conventional dilators are tapered from a proximate (trailing) end or from a point along its length to its distal end for the comfort of the patient. This taper is intended to cause the above-mentioned gradual dilation of the body opening. In these instances, the distal end of the catheter is normally tapered to match the taper of the dilator. This is intended to cause a substantially smooth transition between the distal end of the catheter and a portion of the dilator. As known in the art, the smoother the transition region between the internally positioned dilator and the catheter, the more comfortable the insertion will be for the patient. However, conventional internally positioned dilators cannot create a transition with an externally positioned instrument that is smooth enough that the patient will not feel any discomfort at the transition.

When a conventional catheter and internally positioned dilator are positioned in an area of the body that is very sensitive, such as a nipple during a ductal access procedure, any discontinuity or break in the transition region between the catheter and the internal dilator will cause the patient significant amounts of discomfort. The discomfort caused by the transition region between conventional dilators and catheters can be so significant that the patient will be discouraged from having the procedure performed again. This can lead to serious consequences, especially when the procedure is for diagnostic purposes, such as determining if the patient has precancerous or cancerous cells within her breast ducts or other portions of the body.

European Patent Application EP-A-0093101 discloses a device for introducing a catheter into a blood vessel. The device is only introduced percutaneously.

International PCT Application WO 99/55384 discloses a dual lumen catheter for obtaining material from breast ducts. A series of galactography dilators of increasing size are used to dilate the ductal opening to permit access by the catheter.

### Summary of the Invention

An aspect of the present invention relates to a device as defined in the appended claims for comfortably dilating a body opening and, if needed, a portion of a passageway proximate the opening. In one embodiment of the present invention, the device can be used to comfortably dilate an opening of a nipple duct and a portion of a nipple duct proximate the opening before or during a ductal access procedure.

The present invention includes an expandable medical dilator as defined in the appended claims for dilating an opening of a nipple duct during or in preparation for the performance of a medical procedure. The dilator comprises an outer wall for positioning against an inner surface of the opening as the opening is being dilated and an inner wall defining an internal lumen for receiving a medical instrument being used to perform the medical procedure. The dilator also includes at least one expansion region. In one embodiment, the expansion region extends from the proximal end to the distal end of the dilator. In another embodiment, the expansion region extends from a position along the length of the dilator to the distal end. In either embodiment, the expansion region can include at least one opening or a plurality of spaced perforations.

The device of the present invention may be used in a method of dilating an opening of a nipple duct. The method comprises the steps of positioning the expandable dilator within the body through the opening and introducing a medical instrument into an inner lumen of the expandable dilator. The method also includes the steps of expanding a distal end of the dilator and dilating the opening.

The device of the present invention isolates the transition between the dilator and the inserted instrument from the wall of the opening and/or the lining of the passageway. This isolation prevents the patient from feeling the extreme levels of discomfort associated with the transition regions of conventional dilators and externally applied medical instruments. The dilator according to the present invention also permits larger catheters or catheters with large bulbous heads to be inserted within the body because the transition region is isolated. Larger catheters with larger internal diameters can be beneficial when cell clumps are being collected during procedures such as ductal lavage. These larger catheters will allow for the recovery of large cell clumps and more efficient cell collection in general.

### Brief Description of the Drawings

Fig. 1 illustrates a dilator according to the present invention positioned within a breast duct;
Fig. 2 illustrates a perspective view of a dilator according to an embodiment of the present invention;
Fig. 3 illustrates a second embodiment of a dilator according to the present invention;
Fig. 4 is a side view of a catheter positioned within an expanded dilator according to the present invention;
Fig. 5 is a perspective view of an expanded dilator according to the present invention with the catheter removed;
Fig. 6 is a cross-sectional view of the dilator shown in Fig. 5 taken along the line 5-5;
Fig. 7 illustrates an embodiment of an expanded dilator according to the present invention positioned within a breast duct;
Fig. 8 illustrates another embodiment of a dilator according to the present invention;
Fig. 9 illustrates another embodiment of a dilator according to the present invention;
Fig. 10 illustrates another embodiment of a dilator according to the present invention with a stop;
Fig. 11 illustrates another embodiment of a dilator according to the present invention positioned within a breast duct;
Figs. 12 and 13 illustrate alternative embodiments of the dilator illustrated in Fig. 11;
Fig. 14 is a side view of the dilator illustrated in Fig. 11 including a catheter with an atraumatic end;
Fig. 15 is a top view of the dilators illustrated in Figs. 1 and 11; and
Fig. 16 is a side view of an alternative embodiment of a dilator according to the present invention.

### Detailed Description of the Invention

As illustrated in the figures, the present invention relates to an expandable medical dilator 10 that is externally positioned about an outer surface of a medical instrument, such as a catheter. The external, expandable dilator 10 isolates and prevents the transition between the dilator and medical instrument from contacting any portion of the patient. The external, expandable dilator 10 according to the present invention allows for a substantially, if not completely, painless dilation (gradual expansion) of an opening 1 of a nipple duct and a body passageway 2.

Body passageways are limited to breast ducts. As discussed above, body openings referred to herein are limited to natural openings in the body. Naturally occurring body openings include ductal openings, such as breast duct openings in a nipple.

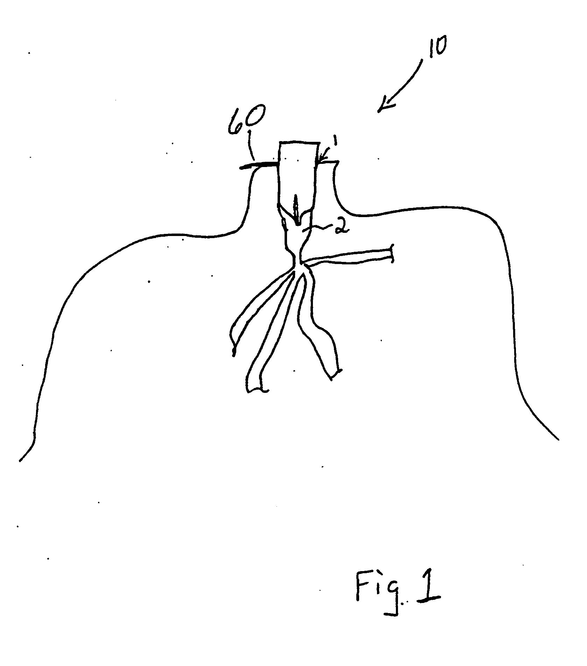

For ease of explanation, the external dilator 10 will be described in conjunction with the introduction of a catheter 5 into a breast duct 2, as shown in Fig. 1, before or during a ductal access procedure, such as ductal lavage, in order to dilate the elastic fibers of the tissue forming the ductal openings within the nipple and the ducts within the breasts. The external dilator 10 according to the present invention is limited to being used with ductal access procedures.

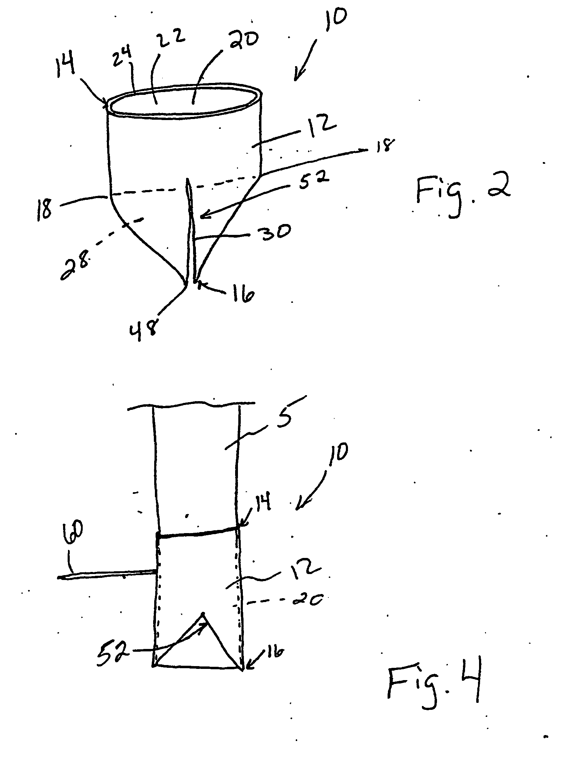

As shown in Fig. 2, the expandable, external dilator 10 includes an outer wall 12 that contacts the body opening 1 and passageway 2 as the external dilator 10 is introduced into the body opening prior to or during the introduction of the catheter 5. The dilator 10 can have a tapered outer profile that makes its introduction into the opening easier and less painful. The outer wall 12 can taper from a proximal end 14 to a distal end (tip) 16. Alternatively, as shown in Fig 2, the outer wall 12 can taper from a position 18 along the length of the external dilator 10 to the distal end 16. The taper of the external dilator 10 can be substantially constant from the proximal end 14 or the position 18 to the distal end 16.

The externally positioned dilator 10 has a length of between about 2cm and about 8cm. In one embodiment, the length of dilator 10 is between about 3cm and about 6cm. In another embodiment, the length of the dilator 10 is about 4cm. The working distance of the dilator 10 can be less than its length. For example, for a 4cm long dilator, the working length is about 2cm.

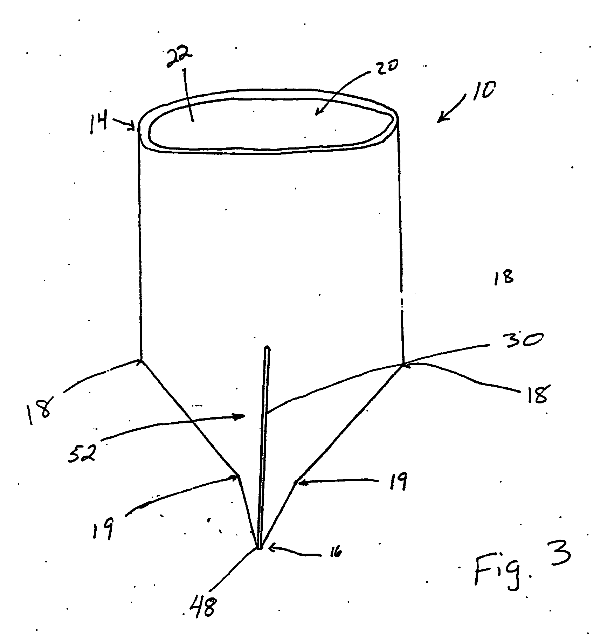

In another embodiment, shown in Fig. 3, the taper is not constant. Instead, the taper begins at either the proximal end 14 or the position 18 and terminates at a position 19 that is short of the distal end 16. The portion 17 of the external dilator 10 between the position 19 and the distal end 16 can have a substantially constant diameter or a second, steeper taper. In this embodiment, portion 17 can have a length of between about 1cm and about 4 cm. In another embodiment, portion 17 has a length of about 2cm. No matter the taper, at least a portion of the external dilator 10 can be expanded so that the internally received catheter 5 can enter proximal end 14 and pass through distal end 16 as discussed below.

In its unexpanded state, the distal end 16 of the catheter 10 has an atraumatic tip 48 as shown in Fig. 2. The atraumatic tip 48 has the tapered shape of a conventional internally positioned dilator or any shape that permits it to be introduced into the body opening without percutaneously entering or otherwise injuring the patient. The unexpanded, distal end 16 of the external dilator 10 is also sized to have the same outer diameter as the distal end of a conventional, internally positioned dilator. For example, the distal end 16 of the dilator 10 has an unexpanded outer diameter of about 0.25mm (0.010 inch) at 5mm and gradually increases to an outer diameter of about 0.89mm (0.035 inch) at about 15mm from the distal end. At about 2cm from the distal end to the proximal end (4cm), the tapered outer diameter of the dilator 10 can increase from about 0.89mm (0.035 inch) (at 2cm) to about 3.16mm (0.125 inch) (at the proximal portion of the cone).

As shown in Fig. 4, unlike conventional dilators, the externally positioned dilator 10 includes an inner lumen 20 that receives and surrounds a portion of the catheter 5. The lumen 20 is defined by an inner surface 22 that extends between an inner edge 24 of proximal end 14 and an inner edge 26 of proximal end 16. The diameter of the inner lumen 20 at the proximal edge 24 is sized to receive the catheter 5. In one embodiment, the inner diameter at the proximal edge 24 is large enough to receive a conventional catheter. Like the outer surface, the inner sidewall 22 tapers from the proximal edge 24 or a position along the length of the inner sidewall 22 to the distal edge 26 or a position spaced from the distal edge 26. The tapered inner sidewall 22 creates an internally tapered region 28 of the external dilator 10 that begins where the inner lumen 20 begins to taper. The tapered region 28 (partially outlined with broken lines) permits the dilator 10 to have an inner diameter that is large enough at the proximal end 14 to receive the catheter 5 and an outer diameter at the distal end 16 that is small enough that the dilator 10 can be positioned in the opening 1 without causing the patient any discomfort.

The inner diameter of the external dilator 10 at the distal edge 26 is essentially negligible when the external dilator 10 is in an unexpanded state as shown in Fig. 2. As the catheter 5 is inserted into the tapered region 28, the tapered region 28 expands and the external dilator 10 assumes an expanded state, as shown in Fig. 4. When this occurs, the internal diameter of the distal end 16 is substantially the same as the outer diameter of the catheter 5. As a result, the outer diameter of the dilator 10 at the distal end 16 is equal to about the sum of the outer diameter of the catheter 5 and the thickness of the dilator wall at the distal end 16. A preferred inner diameter of the expanded lumen 20 at the distal edge 26 is large enough to receive a conventional catheter so that the catheter 5 can extend completely through the external dilator 10 and into the duct. The inner diameter of the lumen 20 will vary with the size of the dilator 10 and the catheter being received.

In order to assume the expanded state illustrated in Fig. 4, the external dilator 10 includes an expansion system having at least one expansion region 52. Each expansion region 52 has at least one longitudinally extending expansion opening 30 and/or at least one tear away (frangible) region 40. The expansion region 52 includes the area occupied by expansion opening(s) 30 and/or the tear away region(s) 40. The expansion system operates to open and expand the dilator 10 and the body opening 1 in response to the introduction and advancement of the catheter 5 within the internal lumen 20 of the dilator 10. The expansion system including the openings 30 and/or the tear away regions 40 cooperate so that the dilator 10 expands to an extent that it can be slid along the catheter 5, peeled away from around the catheter 5 or otherwise removed from within the body after the catheter 5 has been introduced into the body.

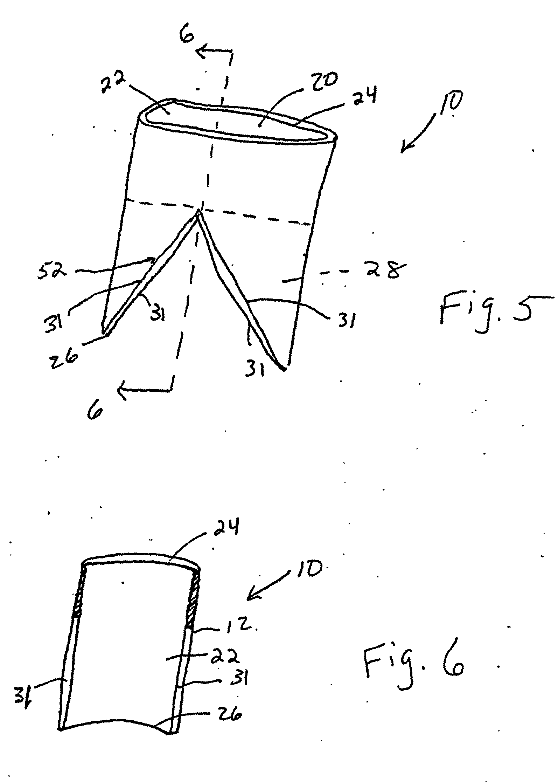

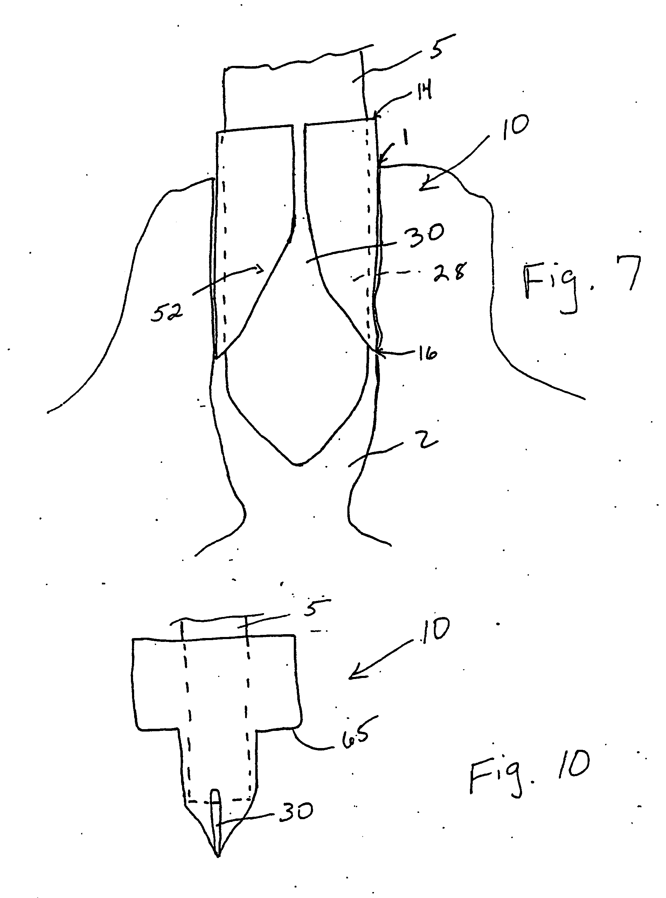

As illustrated in Figs. 2-5, the expansion system 50 includes two longitudinally extending openings 30 (only one shown) that are spaced from each other by 180 degrees. A pair of opposed, circumferentially positioned sidewalls 31 defines each opening 30. The sidewalls 31 extend between the inner sidewall 22 and the outer wall 12 of the dilator 10 so that the dilator 10 can expand as needed. The openings 30 are on opposite sides of the external dilator 10. The openings 30 extend from the distal end 16 to at least the proximal end of the tapered region 28 in order to expand the tapered region 28 in response to the insertion of the catheter 5. The openings 30 can also terminate in the tapered region 28, at a point outside the tapered region 28 at the proximal or extend through the proximal end 14. As shown in Fig. 7, the opening 30 extends from the distal end 16 along the length of the dilator 10 and through the proximal end 14 as discussed below. In alternative embodiments, the external dilator 10 could include three or more openings 30 that are evenly spaced from each other around the circumference of the external dilator 10. For example, when the external dilator 10 includes four openings 30 that are spaced 90 degrees apart from each other around the circumference of the external dilator 10.

As the catheter 5 is introduced into the lumen 20, the sidewalls 31 that define each opening 30 separate and the portion(s) of the external dilator 10 that carry the openings 30 expands into the position shown in Fig. 4. The catheters 5 expands the lumen 20 so that the inner diameter at the proximal edge 24 is substantially the same as the outer diameter of the catheter 5.

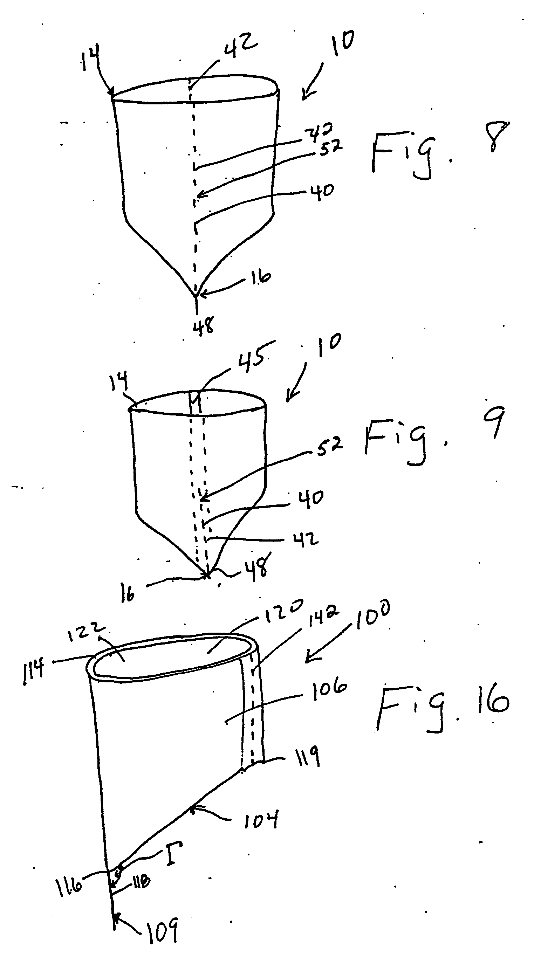

In an alternative embodiment shown in Figs. 8 and 9, the external dilator 10 is similar to that illustrated in Fig. 2. However, in this alternative embodiment, the expansion system 50 includes at least one of the above-mentioned frangible or "tear away" regions 40. The tear away region can be located on one or more sides around the circumference of the dilator 10. The regions can be evenly or randomly spaced relative to each other. Each tear away region 40 includes a row of spaced perforations 42, at least one scored line having a reduced thickness or other known breakable connectors. During the medical operation, as the catheter 5 is advanced through the lumen 20 in the direction of the distal end 16, the external dilator 10 expands and the perforations 42 break along the length of the external dilator 10. As with the other embodiments, the expansion of the external dilator 10 permits the catheter 5 to be positioned within the ductal opening 1 without the patient feeling any discomfort. It is also contemplated that the external dilator 10 could include at least one opening 30 and at least one tear away region 40.

With any of the above-discussed embodiments, when the opening 30 and/or the tear away region 40 extend longitudinally along one side of the dilator 10 and through the proximal end 14, the side of the dilator 10 opposite the opening 30 or tear away region 40 can include a hinge 45 at which the dilator 10 flexes. The hinges can be a function of the flexibility of the material used for the dilator 10 and/or it can include a weakness formed in the internal sidewall of the dilator. The weakness can include a scored inner surface or an area or reduced cross section.

As shown in Figs. 4 and 10, the externally positioned dilator 10 essentially forms a cocoon around the catheter 5 and expands to different sizes in response to the position of the catheter 5 within the lumen 20. Also, the catheter enveloping function of the dilator 10 prevents the catheter 5 from entering the duct until after the dilator 10 has at least partially dilated the opening 1. As discussed above, the outer diameter of the positioned dilator 10 is greater than the outer diameter of the inserted, internally positioned catheter 5. Additionally, because the catheter 5 is received in the dilator 10, the transition between the distal end of the catheter 5 and the dilator 10 is spaced from the lining of the ductal opening by the walls of the dilator 10. For example, as shown in Fig. 7, the distal end of the catheter 5 is spaced from the lining of the dilated ductal opening 1 and duct 2 as a result of the ductal opening expanding to a size that accommodates the larger diameter of the dilator 10. Therefore, the distal end of the catheters does not contact the sidewalls of the opening 1 or the duct 2 and, the patient does not feel any of the discomfort associated with the transition regions of conventional internal dilators. This is especially true when the catheter 5 and dilator 10 are advanced together to the final depth of the catheter 5 and then the dilator 10 is removed from the duct.

In an alternative embodiment, the expansion system can be active, not passive as discussed above, so that the dilator 10 can expand at a rate that is different from that at which the dilator 10 expands when the catheter 5 is advanced through lumen 20. Such a system can be advantageous when a body opening needs to be completely dilated before the catheter 5 is positioned within the lumen 20. In this alternative embodiment, the active expansion system includes a powered expansion mechanism (not illustrated) that is operated by a remotely positioned control system. The powered mechanism can include a plurality of linkages that expand the dilator 10. In another embodiment of the active expansion system, the system can include at least one expandable balloon having a central lumen. The central lumen of the balloon includes a substantially rigid bearing surface that is fixedly secured to the interior surface of lumen 20 and through which the catheter 5 can pass. The balloon could be in fluid communication with a controlled air source positioned outside the patient. In this instance, the air would be delivered through conduits extending within the sidewalls of the dilator 10 or along the inner walls of the lumen 20. The air source could include a manual or automatic pump.

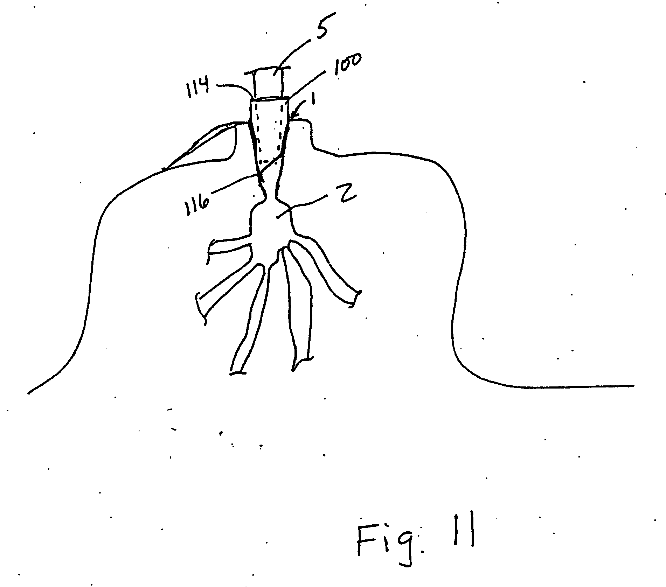

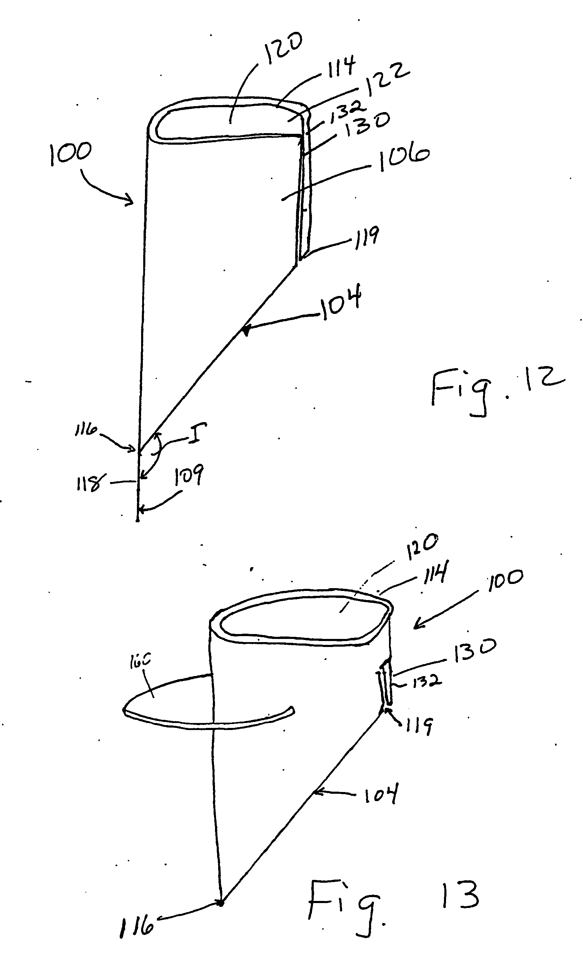

In an embodiment illustrated in Fig. 11, an expandable dilator 100 is positioned about the exterior of a catheter 5 for isolating a transition from between the catheter 5 and the external dilator 100 from the lining of the ductal opening 1. As seen in Fig. 12, the external dilator 100 includes a proximal end 114 and a distal end 116. The external dilator 100 also includes a beveled portion 104 that extends between the distal end 116 and a distal end 108 of a cylindrical portion 106. In an alternative embodiment, the distal end 116 could include a very thin, elongated guiding section 109 as shown in Fig. 12. This elongated guiding section 109 includes a guide element 118, such as a guide wire. In a preferred embodiment, the length of the guide element is controlled based on the estimated distance from the outer surface of the nipple to the sphincter within the breast. It can be advantageous to size the length of the guide member 118 so that it will not contact and penetrate the sphincter. In this alternative embodiment, the beveled portion 104 extends from a proximal end of the guide element 118 to a distal end of the cylindrical portion 106. The cylindrical portion 106 extends between the beveled portion 104 and the proximal end 114 of the catheter 100.

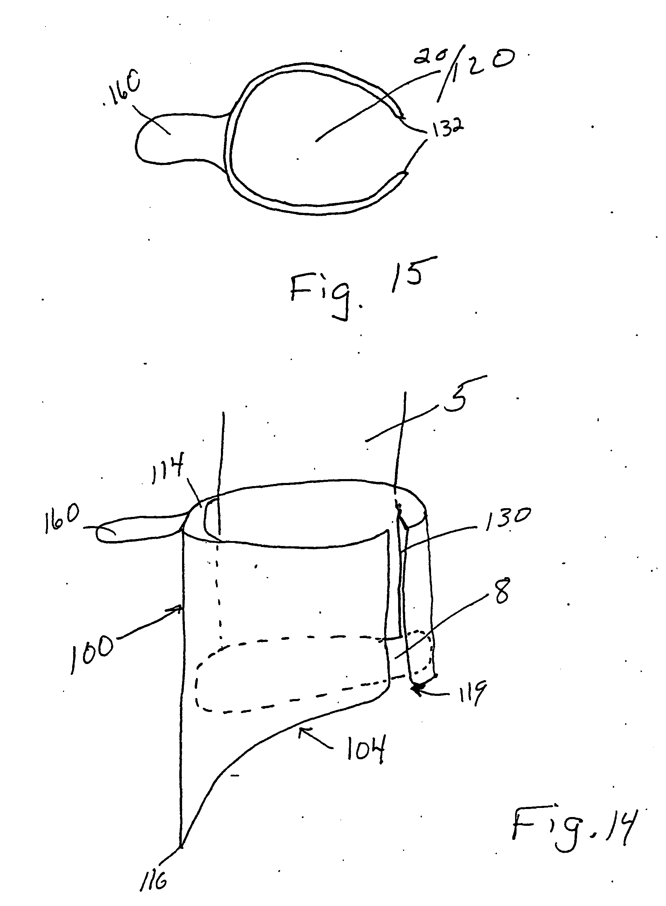

As shown in Fig. 14, the dilator 100 can have a substantially funnel-like shape. This shape allows a catheter with a bulbous or other type of atraumatic tip to be easily introduced into the proximal end 114 of the dilator 100. Also, the enlarged proximal end 114 can act as a stop to prevent the dilator 100 from being lost in the breast duct.

Like external dilator 10, the distal end 116 and the taper of the beveled portion 104 are sized so that the external dilator 100 can be positioned within the opening 1 without the use of any other introduction device. The cylindrical portion 106 has a length between about 1cm and about 5cm. In another embodiment, the cylindrical portion has a length of about 2cm. The total length of the cylindrical portion 106 and the beveled portion 104 is between about 2cm and 8cm. In another embodiment, the total length is about 4cm from the proximal end of the cylindrical portion 106 to the distal end of the beveled portion 104. The cylindrical portion 106 has an outer diameter of about 1mm (0.040 inch) and an inner diameter of about 0.89mm (0.035 inch). However, these sizes will change depending on the size of the catheter being received. In the above-discussed embodiments, the beveled section 104 begins about 2cm above the distal tip and angles 45 degrees or greater to the distal tip. In a preferred embodiment, the distal end is rounded or beveled to reduce trauma to contact tissue.

The dilator 100, shown in Fig. 12, also includes an internal lumen 120 that receives the catheter 5. The internal lumen 120 extends between the proximal end 114 and an outer surface 107 of the beveled portion 104 that forms an angle r with the longitudinal axis of the dilator 100. The internal lumen 120 is defined by at least one internal sidewall 122.

The external dilator 100 can include at;least one longitudinal opening 130 that extends along the length of the external dilator 100. In a first embodiment, the opening 130 extends along the entire length of the cylindrical section 106 of the external dilator 100 from a proximal end 119 of the beveled surface 104 through the - proximal end 114 of the external dilator 100 as shown in Fig. 12. In this embodiment, the dilator 100 may be sized so that the bulbous end of an atraumatic catheter is smaller than the outer diameter of the dilator 100 but larger than the diameter of the inner lumen 120 so that another, non-concentrically positioned catheter cannot be inserted using the same dilator 100 during the same procedure as shown in Fig. 14.

In a second embodiment shown in Fig. 13, the opening 130 extends along only a portion of the cylindrical portion 106 and the length of the dilator 100. The opening 130 in this second embodiment permits the flexing of the dilator for easy removal from the catheter 5 after the catheter 5 is positioned within the duct. In another embodiment, the dilator 100 includes two or more of the above-mentioned openings 130 that are either evenly or randomly spaced from each other around the circumference of the dilator.

The longitudinal opening(s) 130 allows the dilator 100 to expand in response to the internal positioning of the catheter 5. During the medical procedure, the catheter 5 is inserted into the internal lumen 120 and the opposing sidewalls 132 that define the longitudinal boundaries of the opening 130 separate from each other. As a result, the cylindrical section 106 and the beveled portion 104 expand within the breast duct opening 1 in response to the insertion of the catheter 5. The expansion of the dilator 100 causes the breast duct opening 1 to dilate. When this occurs, the internally located catheter 5 is positioned within the ductal opening 1 and spaced from the walls of the duct so that the transition between the catheter 5 and the dilator 100 does not contact the internal lining of the ductal opening 1 and cause the patient any discomfort.

In an alternative embodiment shown in Fig. 16, the external dilator 100 is similar to that illustrated in Fig. 9. However, in this embodiment, the external dilator 100 does not include at least one opening 130 that permits the dilator 100 to expand. Instead, the external dilator 100 includes at least one tear away region 140. The tear away region 140 includes a row of spaced perforations 142, scored sidewalls with a reduced thickness or other known breakable connectors that extend from the proximal end 114 or a position along the cylindrical portion 106 to the distal end of the cylindrical portion 106 of the distal end 116 of the dilator 100. During the medical operation, as the catheter 5 is advanced through the lumen 120 in the direction of the distal end 114, the perforations 142 or scored walls break along the length of the external dilator 100 as the catheter is inserted and the external dilator 100 expands. As with the other embodiments, the expansion of the external dilator 100 opens the duct and permits the catheter 5 to be positioned within the ductal opening 1 without the patient feeling any discomfort.

It is also contemplated that the external dilator 100 could include at least one opening 130 and at least one tear away region 140. As discussed above with respect to the dilator 10, the dilator 100 can include a hinge 145 that extends along the length of the dilator 100 on the opposite side of the dilator 100 from the opening 130 or tear away region 140.

In the above embodiments, the openings 30 and 130 and the tear away regions 40 and 140 permit the expanded dilators 10 and 100 to be slid along the catheter 5, out of the body opening 1 and over the proximal end of the catheter. The tear regions 40 and 140 also permit the dilator to be torn away from the catheter and removed from the duct without having to slide the dilator over the proximal end of the catheter 5. When the dilator 10, 100 includes more than one tear away zone, the pieces of the dilator can be individually removed from around the catheter and out of the body.

In any of the above-mentioned embodiments, the dilators 10, 100 can each include one or more stabilizing members or wings 60, 160, respectively, proximate their proximal edges 14, 114. Each of the stabilizing members 60, 160 extends away from the sidewalls of its respective dilator cylindrical portions. These stabilizing members 60, 160 extend outward away from the body opening 1 so that the dilator 10, 100 will not fall into the ductal opening. Additionally, an underside of the stabilizing member 60, 160 can include an adhesive for adhering to the body around the nipple so that the dilator 10, 100 will not move relative to the opening 1. In an alternative embodiment shown in Fig. 10, the external surface of either dilator 10, 100 could include a step that acts as a stop member to prevent the dilator from being positioned too far within the duct. The stop also prevents the dilator 10, 100 from falling into or being lost in the duct.

Each of the above-mentioned dilators 10, 100 can be formed of any material such as plastics, metals or any other known material that has sufficient column strength to withstand the force of inserting the dilator within a duct without collapsing and sufficient elasticity to flex in response to the introduction of the catheter 5. For example, a metal such as surgical stainless steel can be used. Metals provide the practitioner with the ability to sterilize and reuse the dilators. Alternatively, the dilator could be disposable. In this case, it would be formed of a plastic such as F.E.P. Teflon, polycarbonate, polypropylene, or other known materials.

The device of the present invention may be used in a method of inserting the catheter 5 into the duct. For exemplary purposes, the method will be discussed as it relates to a catheter 5 with an atraumatic head 8 being introduced into the duct 2 using the dilator 100. The catheter 5 is disclosed in copending U.S. Provisional Patent Application to Hung entitled "Medical Instrument with an Atraumatic End".

The nipple is aspirated to determine the location of the fluid producing duct(s). The dilator 100 is positioned over one of the identified ducts and the distal end 116 of the dilator is introduced into the duct. If the dilator 100 includes the guide member 118, then the guide member 118 is first positioned within the duct. As the dilator 100 is gradually advanced into the duct, it dilates the opening to the duct as well as the sidewall lining of the duct 2 itself. When the dilator 100 has been fully inserted, its stabilizing members 160 or the stop member 65 prevent it from going further into the duct than intended.

The catheter 5 can be positioned in the dilator 100 before the dilator 100 is fully seated in the nipple or after it is fully seated. However, when the expandable dilator 10 is used, it may be advantageous to insert the catheter into the dilator 10 after the expanding portion of the dilator has been positioned in the duct.

After the catheter has been deployed into the duct, the dilator 100 is removed from within the duct. However, it is possible to leave the dilator 100 in the duct if the dilator 100 will not interfere with the infusion and collection functions of the catheter 5. Also, the inserted dilator 100 may make the removal of the bulbous end of the catheter more comfortable.

When the dilator 100 is removed, it can be slid up the catheter 5 in the direction of the proximal end 114 and either removed over the proximal end 114 or retained in the vicinity of the proximal end 114 by a connector (adhesive sided material), it can be slid up the catheter 5 and then torn off the catheter 5, it can be slid up the catheter 5 and torn simultaneously or it can be torn and removed in pieces from within the duct. In an alternative embodiment, the dilator 100 can be an integral sliding outer sleeve of the catheter 5 that is secured to the catheter 5 at the proximal end 114 by a flexible connection. In this embodiment, the dilator 100 would merely be slid up the catheter 5 and either partially or completely out of the duct.

Although the foregoing invention has been described in detail by way of illustration and example for purposes of clarity of understanding, it will be readily apparent in light of the teachings of this invention that certain changes and modifications may be made thereto without departing from the scope of the appended claims.

## Claims

1. An expandable medical dilator (10, 100) for dilating an opening (1) of a nipple duct of a patient during or in preparation for the performance of a medical procedure, said dilator comprising an outer wall (12) for positioning against an inner surface of the body opening as the opening is being dilated, an inner wall (22, 122) defining an internal lumen (20, 120) for receiving a medical instrument (5) being used to perform the medical procedure and at least one expansion region (52) to allow the dilator to open and expand, wherein said dilator comprises a proximal end (14, 114) and a distal end (16, 116), and said outer wall tapers from a position along the length of the dilator to the distal end, said distal end has a first outer diameter before the medical instrument is inserted and a second, larger outer diameter after the medical instrument has been introduced at said distal end, and wherein the distal end has an atraumatic tip (48) shaped to permit introduction into the opening of the nipple duct without percutaneously entering the patient.

2. The expandable dilator (10, 100) according to claim 1 wherein said at least one expansion region (52) extends along at least a portion of the length of said dilator.

3. The expandable dilator (10, 100) according to claim 2 wherein each expansion region (52) is defined by at least one opening (30, 130) in said dilator, said at least one opening being defined by opposing wall surfaces (31, 132) that extend between said inner wall (22, 122) and said outer wall (12).

4. The expandable dilator (10, 100) according to claim 2 wherein each expansion region (52) includes at least one tear away region (40, 140).

5. The expandable dilator (10, 100) according to claim 4 wherein each tear away region (40, 140) includes a plurality of spaced perforations (42, 142).

6. The expandable dilator (10, 100) according to claim 3 wherein said at least one opening (30, 130) includes a plurality of openings spaced around the circumference of the dilator.

7. The expandable dilator (10, 100) according to claim 3 wherein said at least one opening (30, 130) extends along the length of said dilator from said proximal end (14, 114) to said distal end (16, 116).

8. The expandable dilator (10, 100) according to claim 3 wherein said at least one opening (30, 130) extends from a position spaced from said proximal end (14, 114) to said distal end (16, 116).

9. The expandable dilator (10, 100) according to claim 1 wherein said inner wall (22, 122) tapers from a position along its length to said distal end (16, 116).

10. The expandable dilator (10, 100) according to claim 1 further including a beveled surface (104) positioned between said proximal end (14, 114) and said distal end (16, 116).

11. The expandable dilator (10, 100) according to claim 10 wherein a portion of said outer wall (12) defines a substantially cylindrical portion (106) of the dilator, and said beveled surface (104) extends between a distal end of said cylindrical portion and said distal end (116) of said dilator.

12. The expandable dilator (10, 100) according to claim 11 wherein said cylindrical portion (106) includes an expansion region (130) extending along at least a portion of its length.

13. The expandable dilator (10, 100) according to claim 12 wherein said expansion region includes at least one opening (130) defined by opposing wall surfaces (132) that extend between said inner (22, 122) and outer walls (12).

14. The expandable dilator (10, 100) according to claim 12 wherein said expansion region includes at least one tear away region (140).

15. The expandable dilator (10, 100) according to claim 14 wherein said tear away region (140) includes a plurality of spaced perforations (142).

16. The expandable dilator (10, 100) according to claim 12 wherein said expansion region extends along said cylindrical portion (106) from the proximal end (14, 114) of the dilator to the distal end of the cylindrical portion (106).

17. The expandable dilator (10, 100) according to claim 10 further including a guide member (109) that extends longitudinally away from said distal end (116).

18. The expandable dilator (10, 100) according to claim 1 further including an expansion region (52) along one side of said internal lumen (20, 120) and a hinge (145) along an opposite side of said internal lumen.

19. The expandable dilator (10, 100) according to claim 1 wherein said dilator further includes a member (60, 160) extending away from a longitudinal axis of the dilator for preventing said dilator from completely entering the body.

20. The expandable dilator (10, 100) according to claim 19 wherein said member (60, 160) includes a wing for securing to a portion of the body.

21. The expandable dilator (10, 100) according to claim 19 wherein said member (60, 160) includes a step along a portion of said outer wall.

22. The expandable dilator (10, 100) according to claim 9 further comprising a stop member (60, 160) for preventing the dilator from extending into the body beyond a predetermined distance.

23. The expandable dilator (10, 100) according to claim 9 wherein said dilator comprises a plurality of expansion regions (52), each said expansion region including an opening (30, 130) defined by opposing wall surfaces (31, 132) that extend between the inner wall (22, 122) and the outer wall (12).

24. The expandable dilator (10, 100) according to claim 9 wherein said dilator comprises a plurality of expansion regions (52) each including a plurality of spaced perforations (42, 142).

25. The expandable dilator (10, 100) according to claim 1 further comprising beveled surface (104) extending at an angle to a longitudinal axis of the dilator.

26. The expandable dilator (10, 100) according to claim 25 further comprising a cylindrical portion (106) extending between said beveled surface (104) and said proximal end (14, 114).

27. The expandable dilator (10, 100) according to claim 26 wherein said expansion region (52) extends along at least a portion of said cylindrical portion (106).

28. The expandable dilator (10, 100) according to claim 27 wherein said expansion region (52) extends from said proximal end (14, 114) to said beveled surface (104).

29. The expandable dilator (10, 100) according to claim 25 further comprising a guiding member (109) extending from said distal end (116) away from said beveled surface (104).

## Patentansprüche

1. Ausdehnbarer medizinischer Dilatator (10, 100) zum Erweitern einer Öffnung (1) eines Brustwarzengangs eines Patienten während oder in Vorbereitung zur Durchführung eines medizinischen Verfahrens, der Dilatator umfasst eine äußere Wand (12) zum Anordnen gegen eine innere Oberfläche der Körperöffnung, wenn die Öffnung erweitert wird, eine innere Wand (22, 122), die ein inneres Lumen (20, 120) zum Aufnehmen eines medizinischen Instruments (5) definiert, das verwendet wird, um das medizinische Verfahren durchzuführen, und mindestens einen Ausdehnungsbereich (52), um zu ermöglichen, dass der Dilatator sich öffnet und ausdehnt, wobei der Dilatator ein proximales Ende (14, 114) und ein distales Ende (16, 116) umfasst, und sich die äußere Wand von einer Position entlang der Länge des Dilatators zum distalen Ende verjüngt, das distale Ende weist einen ersten äußeren Durchmesser bevor das medizinische Instrument eingeführt wird, und einen zweiten größeren äußeren Durchmesser nachdem das Instrument am distalen Ende eingeführt worden ist auf, und wobei das distale Ende eine nicht verletzende Spitze (48) aufweist, die geformt ist, um eine Einführung in die Öffnung des Brustwarzengangs zu ermöglichen, ohne perkutan in den Patienten einzudringen.

2. Ausdehnbarer Dilatator (10, 100) gemäß Anspruch 1, wobei sich mindestens ein Ausdehnungsbereich (52) entlang mindestens eines Teils der Länge des Dilatators erstreckt.

3. Ausdehnbarer Dilatator (10, 100) gemäß Anspruch 2, wobei jeder Ausdehnungsbereich (52) durch mindestens eine Öffnung (30, 130) im Dilatator definiert ist, die mindestens eine Öffnung durch gegenüberliegende Wandoberflächen (31, 132) definiert ist, die sich zwischen der inneren Wand (22, 122) und der äußeren Wand (12) erstrecken.

4. Ausdehnbarer Dilatator (10, 100) gemäß Anspruch 2, wobei jeder Ausdehnungsbereich (52) mindestens einen Abrissbereich (40, 140) einschließt.

5. Ausdehnbarer Dilatator (10, 100) gemäß Anspruch 4, wobei jeder Abrissbereich (40, 140) eine Vielzahl verteilter Perforationen (42, 142) einschließt.

6. Ausdehnbarer Dilatator (10, 100) gemäß Anspruch 3, wobei die mindestens eine Öffnung (30, 130) eine Vielzahl von Öffnungen einschließt, die um den Umfang des Dilatators verteilt sind.

7. Ausdehnbarer Dilatator (10, 100) gemäß Anspruch 3, wobei sich die mindestens eine Öffnung (30, 130) entlang der Länge des Dilatators vom proximalen Ende (14, 114) bis zum distalen Ende (16, 116) erstreckt.

8. Ausdehnbarer Dilatator (10, 100) gemäß Anspruch 3, wobei sich die mindestens eine Öffnung (30, 130) von einer Position erstreckt, die vom proximalen Ende (14, 114) zum distalen Ende (16, 116) verteilt ist.

9. Ausdehnbarer Dilatator (10, 100) gemäß Anspruch 1, wobei sich die innere Wand (22, 122) von einer Position entlang ihrer Länge zum distalen Ende (16, 116) verjüngt.

10. Ausdehnbarer Dilatator (10, 100) gemäß Anspruch 1, der weiter eine schräge Oberfläche (104) einschließt, die zwischen dem proximalen Ende (14, 114) und dem distalen Ende (16, 116) angeordnet ist.

11. Ausdehnbarer Dilatator (10, 100) gemäß Anspruch 10, wobei ein Teil der äußeren Wand (12) einen im Wesentlichen zylindrischen Teil (106) des Dilatators definiert und sich die schräge Oberfläche (104) zwischen einem distalen Ende des zylindrischen Teils und dem distalen Ende (116) des Dilatators erstreckt.

12. Ausdehnbarer Dilatator (10, 100) gemäß Anspruch 11, wobei der zylindrische Teil (106) einen Ausdehnungsbereich (130) einschließt, der sich entlang mindestens eines Teils seiner Länge erstreckt.

13. Ausdehnbarer Dilatator (10, 100) gemäß Anspruch 12, wobei der Ausdehnungsbereich mindestens eine Öffnung (130) einschließt, die durch die gegenüberliegenden Wandoberflächen (132) definiert ist, die sich zwischen der inneren (22, 122) und der äußeren Wand (12) erstreckt.

14. Ausdehnbarer Dilatator (10, 100) gemäß Anspruch 12, wobei der Ausdehnungsbereich mindestens einen Abrissbereich (140) einschließt.

15. Ausdehnbarer Dilatator (10, 100) gemäß Anspruch 14, wobei der Abrissbereich (140) eine Vielzahl verteilter Perforationen (142) einschließt.

16. Ausdehnbarer Dilatator (10, 100) gemäß Anspruch 12, wobei sich der Ausdehnungsbereich entlang dem zylindrischen Teil (106) des proximalen Endes (14, 114) des Dilatators zum distalen Ende des zylindrischen Teils (106) erstreckt.

17. Ausdehnbarer Dilatator (10, 100) gemäß Anspruch 10, der weiter ein Leitelement (109) einschließt, das sich in Längsrichtung vom distalen Ende (116) weg erstreckt.

18. Ausdehnbarer Dilatator (10, 100) gemäß Anspruch 1, der weiter einen Ausdehnungsbereich (52) entlang einer Seite des inneren Lumens (20, 120) und ein Gelenkteil (145) entlang einer gegenüberliegenden Seite des inneren Lumens einschließt.

19. Ausdehnbarer Dilatator (10, 100) gemäß Anspruch 1, wobei der Dilatator weiter ein Element (60, 160) einschließt, das sich von der Längsachse des Dilatators weg erstreckt, um zu verhindern, dass der Dilatator vollständig in den Körper eindringt.

20. Ausdehnbarer Dilatator (10, 100) gemäß Anspruch 19, wobei das Element (60, 160) einen Flügel zum Sichern an einem Teil des Körpers einschließt.

21. Ausdehnbarer Dilatator (10, 100) gemäß Anspruch 19, wobei das Element (60, 160) eine Stufe entlang eines Teils der äußeren Wand einschließt.

22. Ausdehnbarer Dilatator (10, 100) gemäß Anspruch 9, der weiter ein Stoppelement (60, 160) umfasst, um zu verhindern, dass sich der Dilatator in den Körper über eine vorbestimmte Strecke hinaus ausdehnt.

23. Ausdehnbarer Dilatator (10, 100) gemäß Anspruch 9, wobei der Dilatator eine Vielzahl von Ausdehnungsbereichen (52) umfasst, jeder Ausdehnungsbereich eine Öffnung (30, 130) einschließt, die durch gegenüberliegende Wandoberflächen (31, 132) definiert ist, die sich zwischen der inneren Wand (22, 122) und der äußeren Wand (12) erstrecken.

24. Ausdehnbarer Dilatator (10, 100) gemäß Anspruch 9, wobei der Dilatator eine Vielzahl von Ausdehnungsbereichen (52) umfasst, von denen jeder eine Vielzahl verteilter Perforationen (42, 142) einschließt.

25. Ausdehnbarer Dilatator (10, 100) gemäß Anspruch 1, der weiter die schräge Oberfläche (104) umfasst, die sich in einem Winkel zur Längsachse des Dilatators erstreckt.

26. Ausdehnbarer Dilatator (10, 100) gemäß Anspruch 25, der weiter einen zylindrischen Teil (106) umfasst, der sich zwischen der schrägen Oberfläche (104) und dem proximalen Ende (14, 114) erstreckt.

27. Ausdehnbarer Dilatator (10, 100) gemäß Anspruch 26, wobei sich der Ausdehnungsbereich (52) entlang mindestens eines Teils des zylindrischen Teils (106) erstreckt.

28. Ausdehnbarer Dilatator (10, 100) gemäß Anspruch 27, wobei sich der Ausdehnungsbereich (52) vom proximalen Ende (14, 114) zur schrägen Oberfläche (104) erstreckt.

29. Ausdehnbarer Dilatator (10, 100) gemäß Anspruch 25, der weiter ein Leitelement (109) umfasst, das sich vom distalen Ende (116) von der schrägen Oberfläche (104) weg erstreckt.

## Revendications

1. Dilatateur médical extensible (10, 100) servant à dilater une ouverture (1) de canal mamelonnaire chez un patient pendant ou en préparation à la réalisation d'une procédure médicale, ledit dilatateur comprenant une paroi externe (12) pour positionnement contre une surface interne de l'ouverture du corps alors que l'ouverture est dilatée, et une paroi interne (22, 122) définissant une lumière (20, 120) intérieure permettant de recevoir un instrument médical (5) servant à accomplir la procédure médicale et au moins une zone d'expansion (52) pour permettre au dilatateur de s'ouvrir et se déployer, dans lequel ledit dilatateur comprend une extrémité proximale (14,114) et une extrémité distale (16, 116), et dans lequel ladite paroi externe se rétrécit à partir d'une position sur la longueur du dilatateur jusqu'à l'extrémité distale, ladite extrémité distale possède un premier diamètre externe avant que l'instrument médical soit inséré et un deuxième diamètre externe, plus grand, une fois que l'instrument médical a été introduit dans ladite extrémité distale, et dans lequel l'extrémité distale possède une pointe atraumatique (48) formée pour permettre l'introduction du canal mamelonnaire dans l'ouverture sans pénétrer dans le patient de manière percutanée.

2. Dilatateur extensible (10, 100) selon la revendication 1, dans lequel ladite au moins une zone d'expansion (52) s'étend sur au moins une partie de la longueur dudit dilatateur.

3. Dilatateur extensible (10, 100) selon la revendication 2, dans lequel chaque zone d'expansion (52) est définie par au moins une ouverture (30, 130) dans ledit dilatateur, ladite au moins une ouverture étant définie par des surfaces de parois opposées (31, 132) qui s'étendent entre ladite paroi interne (22, 122) et ladite paroi externe (12).

4. Dilatateur extensible (10, 100) selon la revendication 2, dans lequel chaque zone d'expansion (52) comprend au moins une zone détachable (40, 140).

5. Dilatateur extensible (10, 100) selon la revendication 4, dans lequel chaque zone détachable (40, 140) comprend plusieurs perforations espacées (42, 142).

6. Dilatateur extensible (10, 100) selon la revendication 3, dans lequel ladite au moins une ouverture (30, 130) comprend plusieurs ouvertures espacées autour de la circonférence du dilatateur.

7. Dilatateur extensible (10, 100) selon la revendication 3, dans lequel ladite au moins une ouverture (30, 130) s'étend sur la longueur dudit dilatateur de ladite extrémité proximale (14, 114) à ladite extrémité distale (16, 116).

8. Dilatateur extensible (10, 100) selon la revendication 3, dans lequel ladite au moins une ouverture (30, 130) s'étend depuis une position espacée de ladite extrémité proximale (14, 114) à ladite extrémité distale (16, 116).

9. Dilatateur extensible (10, 100) selon la revendication 1, dans lequel ladite paroi interne (22, 122) se rétrécit depuis une position sur sa longueur jusqu'à ladite extrémité distale (16, 116).

10. Dilatateur extensible (10, 100) selon la revendication 1, comprenant en outre une surface biseautée (104) positionnée entre ladite extrémité proximale (14, 114) et ladite extrémité distale (16, 116).

11. Dilatateur extensible (10, 100) selon la revendication 10, dans lequel une partie de ladite paroi externe (12) définit une partie sensiblement cylindrique (106) du dilatateur, et dans lequel ladite surface biseautée (104) s'étend entre une extrémité distale de ladite partie cylindrique et ladite extrémité distale (116) dudit dilatateur.

12. Dilatateur extensible (10, 100) selon la revendication 11, dans lequel ladite partie cylindrique (106) comprend une zone d'expansion (130) s'étendant sur au moins une partie de sa longueur.

13. Dilatateur extensible (10, 100) selon la revendication 12, dans lequel ladite zone d'expansion comprend au moins une ouverture (130) définie par les surfaces de parois (132) opposées qui s'étendent entre ladite paroi interne (22, 122) et ladite paroi externe (12).

14. Dilatateur extensible (10, 100) selon la revendication 12, dans lequel ladite zone d'expansion comprend au moins une zone détachable (140).

15. Dilatateur extensible (10, 100) selon la revendication 14, dans lequel ladite zone détachable (140) comprend plusieurs perforations (142) espacées.

16. Dilatateur extensible (10, 100) selon la revendication 12, dans lequel ladite zone d'expansion s'étend le long de ladite partie cylindrique (106) de l'extrémité proximale (14, 114) du dilatateur à l'extrémité distale de la partie cylindrique (106).

17. Dilatateur extensible (10, 100) selon la revendication 10, comprenant en outre un élément de guidage (109) qui s'étend de manière longitudinale à l'opposé de ladite extrémité distale (116).

18. Dilatateur extensible (10, 100) selon la revendication 1, comprenant en outre une zone d'expansion (52) le long d'un côté de ladite lumière intérieure (20, 120) et une charnière (145) le long d'un côté opposé de ladite lumière intérieure.

19. Dilatateur extensible (10, 100) selon la revendication 1, dans lequel ledit dilatateur comprend en outre un élément (60, 160) s'étendant à l'opposé d'un axe longitudinal du dilatateur pour empêcher ledit dilatateur de pénétrer entièrement à l'intérieur du corps.

20. Dilatateur extensible (10, 100) selon la revendication 19, dans lequel ledit élément (60, 160) comprend une aile pour fixation à une partie du corps.

21. Dilatateur extensible (10, 100) selon la revendication 19, dans lequel ledit élément (60, 160) comprend une marche le long d'une partie de la dite paroi externe.

22. Dilatateur extensible (10, 100) selon la revendication 9, comprenant en outre un élément (60, 160) d'arrêt pour empêcher le dilatateur de se déployer à l'intérieur du corps au-delà d'une distance prédéterminée.

23. Dilatateur extensible (10, 100) selon la revendication 9, dans lequel ledit dilatateur comprend plusieurs zones d'expansion (52), chaque dite zone d'expansion comprenant une ouverture (30, 130) définie par des surfaces de parois (31, 132) opposées qui s'étendent entre la paroi interne (22, 122) et la paroi externe (12).

24. Dilatateur extensible (10, 100) selon la revendication 9, dans lequel ledit dilatateur comprend plusieurs zones d'expansion (52), comprenant chacune plusieurs perforations espacées (42, 142).

25. Dilatateur extensible (10, 100) selon la revendication 1, comprenant en outre une surface biseautée (104) s'étendant selon un angle par rapport à un axe longitudinal du dilatateur.

26. Dilatateur extensible (10, 100) selon la revendication 25, comprenant en outre une partie cylindrique (106) s'étendant entre ladite surface biseautée (104) et ladite extrémité proximale (14, 114).

27. Dilatateur extensible (10, 100) selon la revendication 26, dans lequel ladite zone d'expansion (52) s'étend le long d'au moins une partie de ladite partie cylindrique (106).

28. Dilatateur extensible (10, 100) selon la revendication 27, dans lequel ladite zone d'expansion (52) s'étend de ladite extrémité proximale (14, 114) à ladite surface biseautée (104).

29. Dilatateur extensible (10, 100) selon la revendication 25, comprenant en outre un élément de guidage (109) s'étendant de ladite extrémité distale (116) à l'opposé de ladite surface biseautée (104).
